# EUROPEAN PATENT APPLICATION

(11) **EP 4 751 720 A1**
(43) Date of publication of application: **03.06.2026**
(21) Application number: 25207547.8
(22) Date of filing: 08.10.2025
(51) Int. Cl.: A61K 31/19, A61K 31/421, A61P 25/08, A61K 45/06

(54) **2-PENTADECYL-2-OXAZOLINE IN ASSOCIATION WITH BUTYRIC ACID OR A SALT THEREOF IN THE TREATMENT OF EPILEPSY**

(30) Priority: 27.11.2024 IT 202400026724
(71) Applicant: EPITECH GROUP S.p.A., 20144 Milano (MI) (IT)
(72) Inventor: DELLA VALLE, Maria Federica, I-20144 Milano, ITALY (IT); MARCOLONGO, Gabriele, I-20144 Milano, ITALY (IT)
(74) Representative: Long, Giorgio

(57) **Abstract**

The subject matter of the present invention is the use of 2-Pentadecyl-2-oxazoline (also known as PEA-OXA) in association with butyric acid, in acidic or salified form, in the treatment of epilepsy and associated psychiatric comorbidities (such as social isolation).

In particular, the present invention relates to PEA-OXA for use, in humans and animals, in the prevention and treatment of epileptogenesis and comorbidities associated with epilepsy, such as limited social interaction, wherein PEA-OXA is administered in association or in combination with butyric acid or a salt thereof, wherein said administration is separate, joint, or simultaneous.

## Description

### Field of the invention

The subject matter of the present invention is the use of 2-Pentadecyl-2-oxazoline (also known as PEA-OXA) in association with butyric acid, in acidic or salified form, in the treatment of epilepsy and associated psychiatric comorbidities (such as social isolation) in humans and animals.

### Prior art

Epilepsy is a chronic and complex neurological disorder that affects all age groups with incidence peaks during childhood and in the elderly. It is estimated that about 70 million people worldwide are affected by this disease.

Epilepsy also concerns the veterinary field with significant prevalence among companion animals, dogs and cats in particular.

Despite the progress made in the field of diagnostic techniques and in the study of pathological mechanisms, the triggering causes of epileptogenesis are still unknown.

According to the International League Against Epilepsy (ILAE), epileptic seizures consist of "transient recurrences of signs and/or symptoms due to abnormal, excessive, or synchronous cerebral neuronal activity" that generate a short circuit localized in a specific brain area or spread throughout the brain.

Different types of seizures are distinguished based on the involvement of a more or less large group of nerve cells, also defined as epileptogenic foci, which exhibit abnormal and excessive electrical activity.

Seizures can be generalized tonic-clonic (or grand mal), wherein the entire brain is subjected to an intense abnormal discharge, focal with abnormal discharges involving only a limited part of the brain, or absence of seizures (or petit mal), wherein seizures manifest with altered consciousness, lack of reactivity, and memory lapses.

Although the most common symptoms are spasms and/or convulsions (muscle contraction/relaxation), psychiatric comorbidities are very frequent and represent a serious problem for affected subjects who exhibit mood disorders, psychosis, anxiety, depression and, from the onset due to the unpredictability of seizures, social isolation that, in the most severe cases, can lead to suicide.

It is therefore of fundamental importance to promptly diagnose epileptic pathology and related psychiatric comorbidities in order to identify the most appropriate pharmacological therapy to prevent seizures and to improve the quality of life and mental health of patients.

The drugs in use for the treatment of seizures, today called "anti-seizure" drugs, are intended to prevent the recurrence of epileptic attacks. Their choice depends on the type of seizure and on the characteristics of the patient to be treated.

Anti-seizure drugs have different mechanisms of action which are mainly based on the modulation of the function of neurons by regulating their excitability. In fact, they act by enhancing the action of gamma-aminobutyric acid (GABA) receptors or by inhibiting sodium and calcium channels involved in cerebral hyperexcitability and in the genesis of epileptic seizures.

Currently, several classes of anti-seizure drugs are available: the "classic" or first-generation ones such as valproic acid, carbamazepine, phenytoin and phenobarbital, the latter widely used also in veterinary medicine; second-generation drugs, which include levetiracetam, lamotrigine, oxcarbazepine and topiramate; finally, third-generation drugs such as lacosamide, perampanel and rufinamide, mainly used in combined therapies.

Despite the wide range of drugs made available by the scientific community and the good success of therapies for the management of epilepsy and comorbidities, a percentage of treated patients (between 15% and 37%) turn out to be refractory to anti-seizure drugs. We therefore speak of drug-resistant epilepsies, wherein epileptic seizures and psychiatric comorbidities, such as social isolation, persist even in the presence of the combination of two well-tolerated drugs administered at the maximum dose.

When anti-seizure drugs fail, alternative therapies may come into play, such as surgery or the administration of innovative drugs such as cenobamate.

Although anti-seizure drugs are essential for the management of epilepsy, they are not free from side effects which may negatively affect, even more than seizures themselves, the quality of life of the patient. The major adverse effects are of a depressive nature, but secondary effects such as fatigue, drowsiness, nausea and vomiting, dizziness, agitation and nervousness, uncontrollable tremor, speech and memory disorders and, in the most severe cases, hepatic toxicity, may also occur.

For these reasons, there remains an urgent need to develop new therapies free from side effects to prevent epileptogenesis and comorbidities and to counteract, in a more effective and safer manner, epileptic seizures.

In this context, the Applicant has found that the association between the molecule 2-Pentadecyl-2-oxazoline, or PEA-OXA, and butyric acid, in acidic or salified form (one of the most important short-chain fatty acids, physiologically produced by the fermentation of dietary fibres by the colonic intestinal microbiota), has proved to be synergistically active in the prevention and treatment of epileptogenesis and comorbidities associated with epilepsy.

The PEA-OXA molecule, although functioning in part as a precursor of PEA, possesses different and entirely peculiar effects and mechanisms. In particular, unlike PEA, PEA-OXA has been shown to act on neuropsychiatric symptoms, such as depression, through specific interactions with adrenergic α2 and histaminergic H3 receptors [S. Boccella, et al. 2-Pentadecyl-2-oxazoline ameliorates memory impairment and depression-like behaviour in neuropathic mice: possible role of adrenergic alpha2- and H3 histamine autoreceptors, Mol. Brain. 14 (2021) 28; S. Boccella, et al. Treatment with 2-pentadecyl-2-oxazoline restores mild traumatic brain injury-induced sensorial and neuropsychiatric dysfunctions, Front. Pharmacol. 11 (2020) 91], and has also been shown to be specifically capable of inhibiting the TLR4/MD-2 receptor complex (Facci L et al. 2-Pentadecyl-2-oxazoline inhibits lipopolysaccharide-induced microglia activation interfering with TLR4 signaling. Life Sci. 2023:122242).

### Summary of the invention

The present invention derives from the surprising discovery that PEA-OXA, when administered in association with butyric acid in acidic or salified form, such as sodium salt (NaB), exhibits a synergistically relevant effect in the prevention and treatment of epileptogenesis and social isolation.

The present invention therefore relates to PEA-OXA for use in the prevention and treatment of epileptogenesis and comorbidities associated with epilepsy, wherein PEA-OXA is administered in association or in combination with butyric acid or a salt thereof, wherein said administration is separate, joint, or simultaneous.

A particular object of the invention is PEA-OXA for use in the reduction of the severity of epileptic seizures, wherein PEA-OXA is administered in association or in combination with butyric acid or a salt thereof, wherein said administration is separate, joint, or simultaneous.

The invention therefore makes it possible to provide an innovative treatment with a positive impact on the quality of life and mental health of the epileptic subject.

A further advantage of the present invention is the synergistic effect between PEA-OXA and butyric acid or salt thereof, specifically NaB, in the treatment of epilepsy, in humans and animals, which makes it possible to enhance the effects of the individual molecules.

These and further objects, as outlined in the annexed claims, will be described in the following description. The text of the claims must be considered as included in the description for the purposes of assessing sufficiency of disclosure.

Further features and advantages of the invention will result from the description set out below of preferred embodiments, given by way of example and not limitation.

### Brief description of the figures

Figure 1 shows a graph representing the effect over time of NaB and PEA-OXA, used individually or in association, on the severity of epileptic seizure. **p < 0.001 and ***p < 0.0001 PEA-OXA+NaB vs vehicle; °°p < 0.01 and °°°p < 0.0001 PEA-OXA+NaB vs NaB; ^{#}p < 0.05, ^{##}p < 0.01, ^{###}p < 0.0001 PEA-OXA+NaB vs PEA-OXA.
Figure 2 is a graphical representation of the synergistic effect of the association PEA-OXA+NaB on the severity of epileptic seizures at the sixth PTZ injection.
Figure 3 shows a graph representing the effect of NaB and PEA-OXA, used individually or in association, on the time (sec) of interaction of mice with a conspecific. *p<0.0001 vs control; ^{$}p<0.0001 vs vehicle; °p < 0.0001 vs NaB; ^{#}p< 0.0001 vs PEA-OXA.
Figure 4 is a graphical representation of the synergistic effect of the association PEA-OXA+NaB on the time spent by epileptic mice interacting with an unfamiliar mouse.
Figure 5 shows a graph representing the effect of NaB and PEA-OXA, used individually or in association, on the time (sec) spent by mice sniffing an unfamiliar conspecific (sniffing time).

*p<0.0001 vs control; ^{$}p = 0.0002 vs vehicle; °p < 0.0001 vs NaB; ^{#}p =0.0037 vs PEA-OXA.

Figure 6 is a graphical representation of the synergistic effect of the association PEA-OXA+NaB on the time spent by epileptic mice sniffing an unfamiliar mouse.

### Detailed description of the invention

The present invention relates to PEA-OXA for use in the prevention and treatment of epileptogenesis and comorbidities associated with epilepsy, wherein PEA-OXA is administered in association or in combination with butyric acid or a salt thereof, wherein said administration is separate, joint, or simultaneous.

By the terms "in association" or "in combination" it is meant both a combination therapy and a therapy wherein PEA-OXA and butyric acid or a salt thereof are contained in a single dosage form.

By "separate" administration it is meant an administration of PEA-OXA and butyric acid or a salt thereof, administered at different times which may range from 1 minute to several hours, for example at 8, 12 or 24 hours apart from one another.

By "joint" administration it is meant an administration of PEA-OXA and butyric acid or a salt thereof contained in a single dosage form, that is, a pharmaceutical or veterinary composition or formulation.

By "simultaneous" administration it is meant an administration of PEA-OXA and butyric acid or a salt thereof in separate dosage forms, but administered simultaneously, namely within a time interval between the administration of PEA-OXA and that of the butyric acid or a salt thereof, or vice versa, not exceeding 1 minute.

In a particular embodiment, the present invention relates to PEA-OXA for use in the management of epileptic seizures, wherein PEA-OXA is administered in association or in combination with butyric acid or a salt thereof, wherein said administration is separate, joint, or simultaneous.

A further object of the present invention is a composition comprising PEA-OXA and butyric acid or a salt thereof. Preferably, the composition of the invention consists of a dry mixture of PEA-OXA/butyric acid or a salt thereof.

Whether administered separately or jointly in a single formulation, PEA-OXA and butyric acid or a salt thereof are administered in a butyric acid or salt thereof/PEA-OXA weight ratio between 1:1 and 7:1, preferably between 2:1 and 5:1, more preferably between 2.5:1 and 3.5:1, even more preferably of about 3:1.

On the basis of said weight ratios, for which a remarkable therapeutic effect has been demonstrated, the daily dose of PEA-OXA, whether in a combination therapy or in a composition of PEA-OXA with butyric acid or a salt thereof, will be at least between 100 mg/day and 1500 mg/day, preferably between 200 mg/day and 1200 mg/day.

The preferred daily dose of butyric acid or a salt thereof, whether in a combination therapy or in a composition of PEA-OXA with butyric acid or a salt thereof, will be at least between 300 mg/day and 3500 mg/day, more preferably between 400 mg/day and 3000 mg/day.

The above-mentioned doses may be divided into 2-3 daily administrations.

Such doses may vary depending on the subject and in particular whether the subject is in childhood, adulthood, or old age. For example, for paediatric use, average administrations of butyric acid or a salt thereof of 450 mg/day and of PEA-OXA of 300 mg/day may be provided, divisible into 2-3 administrations.

It will nevertheless be possible to use doses of PEA-OXA higher than those described above.

Such daily doses may be divided into dose units for administration, for example, from 1 to 4 times per day. The dose will also depend on the chosen route of administration. It should be considered that variations of the dosage may need to be arranged depending on the age and weight of the patient and also on the extent of the pathology to be treated. The exact dose and the route of administration will finally be at the discretion of the attending physician.

When butyric acid is in the form of a salt, said salt will preferably be selected from sodium salt, potassium salt, calcium salt, magnesium salt, lysine salt, arginine salt or combinations thereof.

In certain embodiments, the composition of the invention further comprises palmitoylethanolamide (PEA). When the composition comprises PEA, it will be present in amounts between 100 mg and 1500 mg per dose unit, preferably between 200 mg and 800 mg per dose unit. PEA may be in non-micronized, micronized or ultra-micronized form, that is with a d90 smaller than 6 microns (measured by laser scattering).

For the purposes of the invention, PEA-OXA alone, butyric acid or a salt thereof alone, or the composition containing PEA-OXA and butyric acid or a salt thereof may be included in pharmaceutical or veterinary formulations and may be formulated in dosage forms for oral, buccal, parenteral, rectal, topical, or transdermal administration.

For oral administration, the compounds of the invention may be found, for example, in the form of tablets or capsules, hard or soft, prepared in the conventional manner with pharmaceutically acceptable excipients such as binding agents (for example pregelatinized maize starch, polyvinylpyrrolidone or hydroxypropyl methylcellulose); fillers (for example lactose, microcrystalline cellulose or calcium hydrogen phosphate); lubricants (for example magnesium stearate, talc or silica); disintegrants (for example potato starch or sodium starch glycolate); or inhibiting agents (for example sodium lauryl sulfate). The tablets may be coated with the methods well known in the art. Liquid preparations for oral administration may be in the form, for example, of solutions, syrups or suspensions or they may be presented as lyophilized products or granulates to be reconstituted, before use, with water or other suitable vehicles. Such liquid preparations may be prepared by conventional methods with pharmaceutically acceptable additives such as suspension agents (for example sorbitol syrup, cellulose derivatives or edible hydrogenated fats); emulsifying agents (for example lecithin or acacia); non-aqueous vehicles (for example almond oil, oily esters, ethyl alcohol or fractionated vegetable oils); and preservatives (for example methyl- or propyl-p-hydroxybenzoates, sorbic acid, benzoic acid or their salts). The preparation may also suitably contain flavourings, colourings, and sweetening agents.

Preparations for oral administration may be suitably formulated to allow controlled release of the active ingredient.

For buccal administration, the compounds of the invention may be used in the form of tablets or granules formulated in the conventional manner, suitable for absorption at the level of the buccal mucosa. Typical buccal formulations are tablets for sublingual administration.

The compounds of the invention may be formulated for parenteral administration by injection. The formulations for injections may be presented in the form of a single dose, for example in ampoules, with an added preservative. The compositions may be in such a form as suspensions, solutions or emulsions in oily or aqueous vehicles and may contain formulary agents such as suspension agents, stabilizers and/or dispersants. Alternatively, the active ingredient or the mixture of active ingredients may be in the form of a powder to be reconstituted, before use, with a suitable vehicle, for example with sterile water.

The compounds of the invention may also be formulated according to rectal formulations such as suppositories or retention enemas, for example containing the basic components of common suppositories such as cocoa butter or other glycerides.

In addition to the formulations described above, the compounds of the invention may also be formulated as depot preparations for administration over a period of one day up to one week. Such long-acting formulations may be administered by implant (for example subcutaneously, transcutaneously, or intramuscularly) or by intramuscular injection. Thus, for example, the composition may be formulated with suitable polymeric or hydrophobic materials (for example in the form of an emulsion in a suitable oil) or ion exchange resins or as minimally soluble derivatives.

The compounds or the composition of the invention may also be administered in the form of oral sprays or nasal sprays.

A further object of the invention are also dietary compositions, food supplements, complementary feed, and foods for special medical purposes (FSMP) comprising PEA-OXA and butyric acid or a salt thereof.

By the term "foods for special medical purposes" it is meant products authorized according to regulation (EU) 2016/128. This term refers to a product to be administered under medical supervision, thus assimilating said FSMP to a drug.

The formulations according to the invention may be prepared according to conventional methods, such as those described in Remington's Pharmaceutical Sciences Handbook, Mack Pub. Co., N.Y., USA, 17th edition, 1985 or in Remington, The Science and Practice of Pharmacy, Edited by Allen, Loyd V., Jr, 22nd edition, 2012 or in subsequent editions.

### EXPERIMENTAL PART

### BIOLOGICAL EXPERIMENTATION

For the in vivo experimentation, healthy male C57BL/6J (C57) mice (Charles River Laboratories Italia s.r.l.) aged 28-34 days were used, fed ad libitum and housed in cages with a controlled sleep/wake cycle. Before the beginning of the experimentation, the animals were subjected to a 1-week acclimatization period, considering all procedures and experimental protocols in compliance with the principles of care and welfare of laboratory animals approved by the Italian Ministry of Health (D.Lgs 2014/26) and by the European directives (EU Directive 2010/63).

The epilepsy model was induced with subcutaneous (s.c.) injections, on alternate days, of Pentylenetetrazole (PTZ, 30 mg/kg), in order to induce the progressive development of kindling (Russo E et al. Lamotrigine positively affects the development of psychiatric comorbidity in epileptic animals, while psychiatric comorbidity aggravates seizures. Epilepsy 2013; Behav 28: 232-240; De Sarro G et al. Seizure susceptibility to various convulsant stimuli of knockout interleukin-6 mice. Pharmacol Biochem Behav 2004; 77: 761-6).

By kindling it is meant a state of progressive and permanent reduction of the convulsive threshold, characterized by the development of generalized tonic-clonic seizures, due to the increasing recruitment of previously non-hyperexcitable neurons. Kindling is considered developed when the tonic-clonic convulsions manifested by the animal reach for three consecutive times the maximum score on the Velisková scale (Velisková J et al. Ketamine suppresses both bicuculline- and picrotoxin-induced generalized tonic-clonic seizures during ontogenesis. Pharmacol Biochem Behav. 1990; 37: 667-74).

The epileptic animals were treated orally with vehicle (1% carboxymethylcellulose, CMC) or with PEA-OXA (20 mg/kg) and sodium butyrate (NaB) (30 mg/kg), used individually or in association with one another, starting seven days before the induction of kindling until the end of behavioural tests.

The control group received by s.c. route the PTZ vehicle (saline solution) and orally the vehicle of the tested molecules (1% CMC).

### Intensity of epileptic seizures

To monitor the intensity of epileptic seizures, mice were kept under observation for 120 minutes after each single injection of PTZ. The intensity of epileptic seizures was measured with the Velisková scale, a validated score from 0 to 5, wherein: 0 = no change in behaviour; 0.5 = abnormal behaviour (sniffing, excessive self-grooming, orientation); 1 = isolated myoclonic jerks; 2 = atypical clonic seizures; 3 = fully developed bilateral clonic contraction of the hind limbs; 3.5 = tonic/clonic contraction of the hind limbs with body twisting; 4 = clonic contraction of all limbs; 5 = full development of tonic-clonic convulsions.

### Social isolation

To evaluate the extent of social isolation induced by epileptic seizures, the interaction time between animals was measured using the three-chamber social test. Briefly, the animals are placed inside a transparent polycarbonate arena, divided into three chambers (one central and two lateral), by means of movable partition walls. In the first 10 minutes, the dividers are left open and the animal is free to explore the three empty chambers. In the second phase, the dividers are closed and two small cages are placed, one in each lateral chamber, containing respectively an unfamiliar adult mouse and an inanimate object. The dividers are then opened allowing the mouse to move freely for 10 minutes in all three compartments of the apparatus.

In the last 10 minutes of the test, the sociability index is evaluated by measuring, thanks to a camera coupled to software, the time spent by the mouse in the chamber containing the unfamiliar mouse and that spent in the chamber containing the inanimate object. During the same session, the time spent by the animal sniffing the unfamiliar mouse or the inanimate object is also evaluated (Tallarico M et al. Seizure susceptibility to various convulsant stimuli in the BTBR mouse model of autism spectrum disorders. Front Pharmacol. 2023: 14: 1155729).

### Statistical analysis

The statistical analysis relied on the Generalized Linear Model (GLM). Comparisons between the means of the individual treatments were evaluated with a post-hoc analysis based on the Tukey-Kramer correction for multiple comparisons.

In order to verify synergy (under the hypothesis that the effect of the combined administration of PEA-OXA and NaB would be greater than the sum of the effects of the two treatments used individually), the treatment groups, excluding the control group, were analysed through a two-way factorial analysis of variance (2x2 ANOVA) according to the procedure illustrated by Slinker BK. The statistics of synergism. J Mol Cell Cardiol. 1998; 30: 723-31.

The results of the analyses are expressed as mean ± standard error of the mean (SEM). The analyses were performed using SAS software, version 9.4 (SAS Institute, Cary, NC, USA).

The value p < 0.05 was considered significant.

### RESULTS OF THE EXPERIMENTS

### Intensity of epileptic seizure

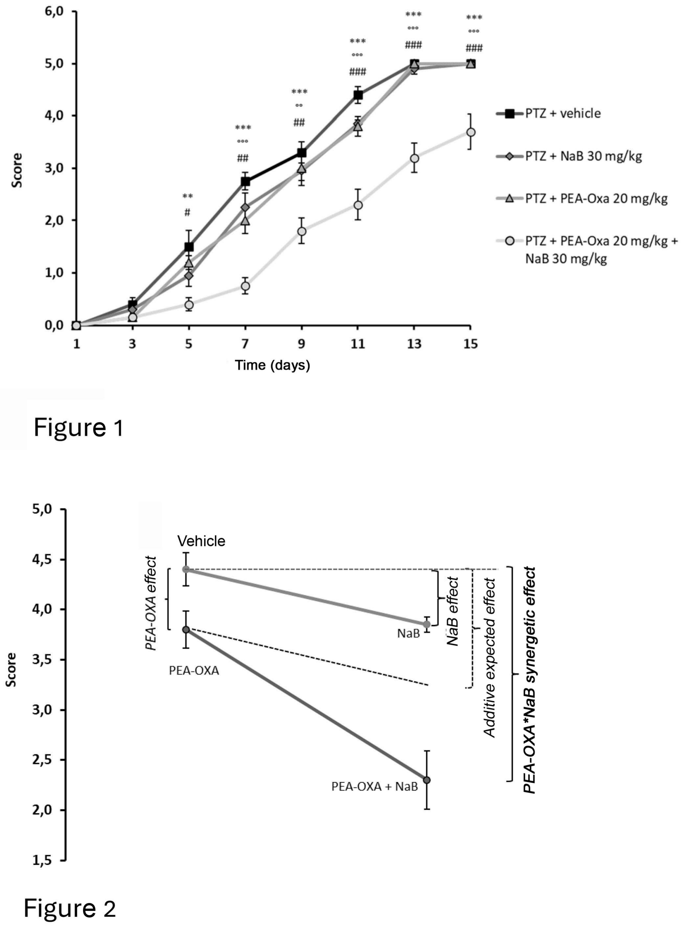

As reported in Fig. 1, the development of epileptic seizure was progressive and directly proportional to the number of PTZ administrations.

Single treatment with (a) vehicle, (b) NaB 30 mg/kg or (c) PEA-OXA 20 mg/kg was not effective in reducing the severity of epileptic seizures (p > 0.05).

The combined use of PEA-OXA 20 mg/kg + NaB 30 mg/kg, instead, allowed a significant and surprising reduction of the severity of epileptic seizure, already starting from the third PTZ injection (day 5), compared with the group treated with vehicle alone, NaB, and PEA-OXA.

As illustrated in Fig. 2, at the sixth PTZ injection (day 11) - that is, when in the control group a kindling of degree higher than 4 (out of a maximum of 5 of the Velisková scale) occurs - the sum of the effects exerted by the individual compounds (expected additive effect, dashed bracket) is lower than the effect exerted by the combined use of the two molecules (large bracket). Graphically, the synergistic effect exerted by the combination PEA-OXA + NaB is immediately appreciable from the fact that the two segments (blue and red) are not parallel to one another, but divergent.

The synergistic effect between PEA-OXA and NaB was also confirmed at the seventh (day 13) and eighth (day 15) PTZ administrations.

The results of the 2x2 ANOVA analysis are reported in Tables 1A, 1B and 1C. The Pr > F value relative to the row PEA-OXA*NaB represents the probability that the effect of the combination is greater than the sum of the effects of the individual substances only by chance. As can be seen, this is a very low probability and lower than the limit p < 0.05, demonstrating a significant synergistic effect between PEA-OXA and NaB.

**Tab. 1A: Results obtained from two-way factorial analysis of variance (2x2 ANOVA) on kindling at the sixth PTZ injection (day 11 of Fig. 1). Data are graphically represented in Fig. 2.**

| | Source | DF | Sum of Squares | Mean Squares | F Value | Pr > F | |
|---|---|---|---|---|---|---|---|
| | NaB | 1 | 10,51 | 10,51 | 27,76 | | <0.0001 |
| | PEA-Oxa | 1 | 11,56 | 11,56 | 30,53 | | <0.0001 |
| **PEA-Oxa*NaB** | | **1** | **2,26** | **2,26** | **5,96** | | **0,0197** |
| | Error | 36 | 13, 63 | 0,38 | | | |

**Tab. 1B: Results obtained from factorial analysis (2x2 ANOVA) on kindling at the seventh PTZ injection (day 13 of Fig. 1).**

| | Source | DF | Sum of Squares | Mean Squares | F Value | Pr > F |
|---|---|---|---|---|---|---|
| | NaB | 1 | 9,03 | 9,03 | 40, 61 | <0.0001 |
| | PEA-Oxa | 1 | 7,23 | 7,23 | 32,51 | <0.0001 |
| **PEA-Oxa*NaB** | | **1** | **7,23** | **7,23** | **32,51** | **<0.0001** |
| | Error | 36 | 8,00 | 0,22 | | |

**Tab. 1C: Results obtained from factorial analysis (2x2 ANOVA) on kindling at the eighth PTZ injection (day 15 of Fig. 1).**

| | Source | DF | Sum of Squares | Mean Squares | F Value | Pr > F |
|---|---|---|---|---|---|---|
| | NaB | 1 | 4,23 | 4,23 | 15,06 | 0,0004 |
| | PEA-Oxa | 1 | 4,23 | 4,23 | 15,06 | 0, 0004 |
| **PEA-Oxa*NaB** | | **1** | **4,23** | **4,23** | **15,06** | **0,0004** |
| | Error | 36 | 10,10 | 0,28 | | |

### Social isolation

### Interaction time

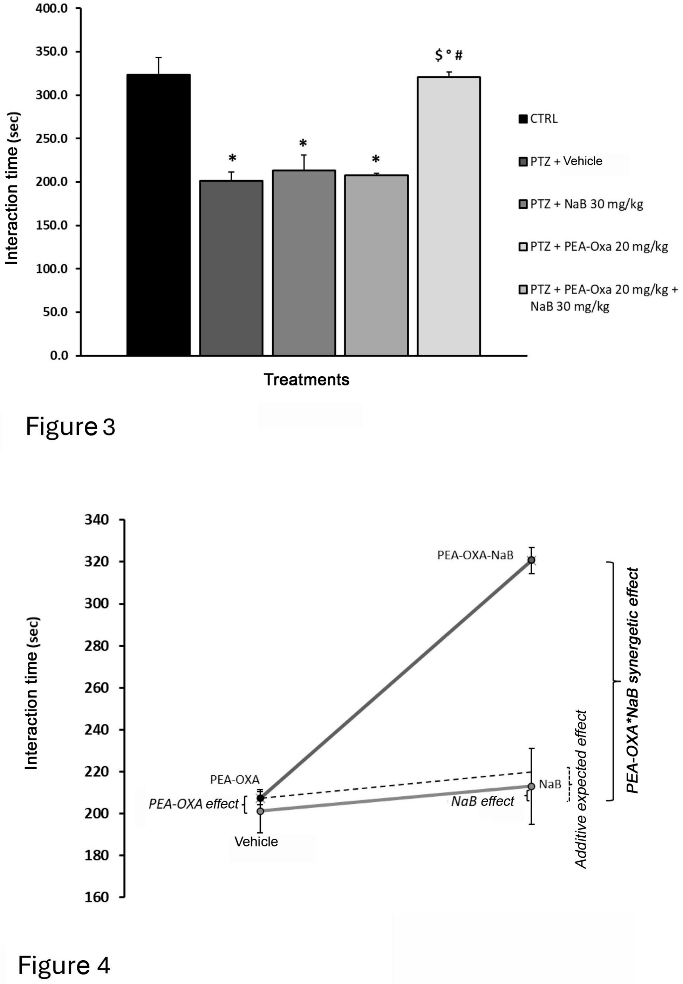

As reported in Fig. 3, PTZ injections drastically reduce the time that epileptic mice spend interacting with a conspecific (p<0.0001).

The groups of animals treated with NaB 30 mg/kg or with PEA-OXA 20 mg/kg do not show significant differences compared with the group treated with vehicle (p = 0.9678 and p = 0.9973, respectively).

The association of PEA-OXA 20 mg/kg and NaB 30 mg/kg significantly increases the interaction time of epileptic mice compared with the vehicle group, as well as compared with the group treated with NaB alone or with PEA-OXA alone (p < 0.0001 for all comparisons).

The interaction time observed after the combined use of the two molecules is not significantly different (p = 0.9999) compared with the control group, demonstrating that the association PEA-OXA + NaB makes it possible to normalize social behaviour, which is strongly altered by epileptic seizures.

As illustrated in Fig. 4, the expected additive effect of the individual compounds (dashed bracket) is clearly lower than the effect exerted by the combined use of the two molecules (large bracket).

Graphically, the synergistic effect exerted by the combination PEA-OXA + NaB is immediately appreciable from the fact that the two segments are not parallel to one another, but divergent.

The results of the 2x2 ANOVA analysis are reported in Tab. 2. As can be seen from the Pr > F value relative to the row PEA-OXA*NaB (for explanation, refer to Tab. 1), the analysis confirmed a significant synergistic effect between PEA-OXA and NaB.

**Tab. 2 - Results obtained from two-way factorial analysis of variance (2x2 ANOVA) performed on the data graphically represented in Fig. 4.**

| Source | DF | Sum of Squares | Mean Squares | F Value | Pr > F |
|---|---|---|---|---|---|
| NaB | 1 | 31387,7 | 31387,7 | 32,70 | <0,0001 |
| PEA-Oxa | 1 | 25900,9 | 25900,9 | 26,99 | <0,0001 |
| **PEA-Oxa*NaB** | **1** | **20584,2** | **20584,2** | **21,45** | **<0,0001** |
| Error | 28 | 26874,2 | 959,8 | | |

### Sniffing

PTZ injections (vehicle group) drastically reduce the time that epileptic mice spend sniffing a conspecific (sniffing time) compared with control animals (p<0.0001).

Unlike the groups treated with NaB 30 mg/kg or with PEA-OXA 20 mg/kg, which do not show significant differences compared with the vehicle group (p = 0.9589 and p = 0.8675, respectively), the association PEA-OXA 20 mg/kg + NaB 30 mg/kg significantly increases sniffing time (p<0.005 for all comparisons; for individual significances refer to the figure description).

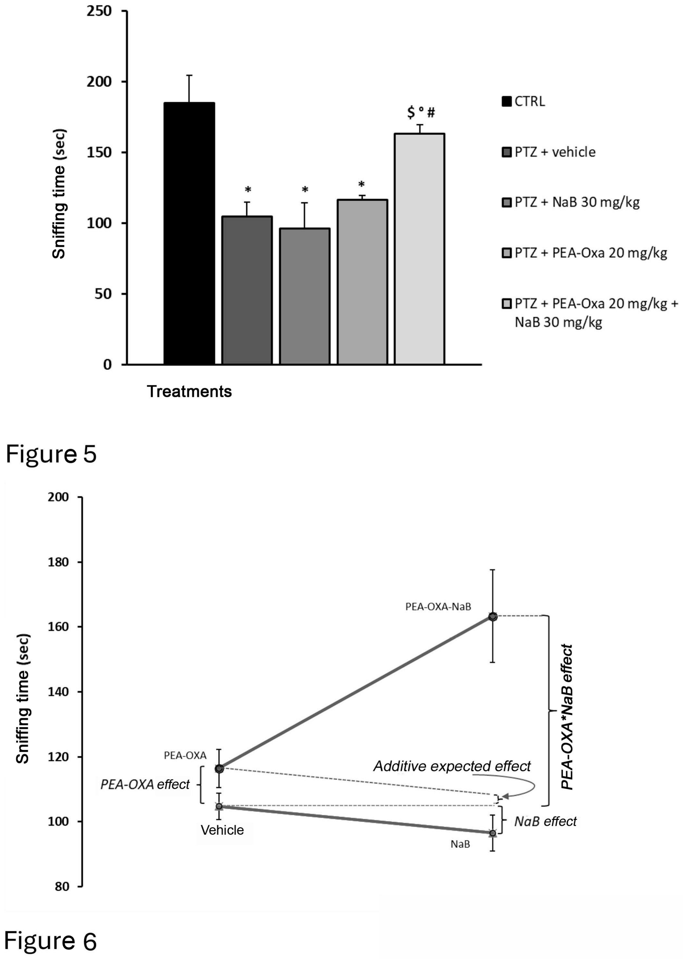

Furthermore, the sniffing time observed in the group treated with this association is not significantly different from that observed in the control group (p = 0.4044), demonstrating that the combined use of PEA-OXA and NaB makes it possible to normalize this exploratory behaviour, significantly impaired by epileptic seizures (Fig. 5).

As illustrated in Fig. 6, the expected additive effect of the individual compounds (dashed bracket) is lower than the effect exerted by the combined use of the two molecules (large bracket). The segments are not only divergent, but have opposite slopes.

Similarly to what emerged for epileptic seizures and interaction time, also in this case the factorial 2x2 ANOVA analysis confirms the synergistic effect between PEA-OXA and NaB (Tab. 3).

**Tab. 3 - Results obtained from factorial analysis (2x2 ANOVA) on sniffing time. Data are graphically represented in Fig. 6.**

| Source | DF | Sum of Squares | Mean Squares | F Value | Pr > F |
|---|---|---|---|---|---|
| NaB | 1 | 2991,5 | 2991,5 | 5,21 | 0,0302 |
| PEA-Oxa | 1 | 12332,4 | 12332,4 | 21,48 | <0,0001 |
| **PEA-Oxa*NaB** | **1** | **6083,6** | **6083,6** | **10,60** | **0,0030** |
| Error | 28 | 16073, 8 | 574,1 | | |

The invention will now be further described by means of the following formulation examples.

### Formulation examples

PEA-OXA = 2-pentadecyl -2-oxazoline

### Example 1 - Delayed-release tablet

| | |
|---|---|
| PEA-OXA | 400 mg |
| Sodium Butyrate | 600 mg |
| Glyceryl Palmitostearate | 200 mg |
| Silica | 20 mg |
| Magnesium stearate | 10 mg |
| Polysorbate 80 | 50 mg |
| Gastroresistant coating | 60 mg |

### Example 2 - Delayed-release tablet for paediatric use

| | |
|---|---|
| PEA-OXA | 100 mg |
| Sodium Butyrate | 150 mg |
| Glyceryl Palmitostearate | 80 mg |
| Silica | 10 mg |
| Magnesium stearate | 5 mg |
| Polysorbate 80 | 10 mg |
| Gastroresistant coating | 20 mg |

### Example 3 - Effervescent tablet

| | |
|---|---|
| PEA-OXA | 600 mg |
| Calcium Butyrate | 900 mg |
| Glyceryl Palmitostearate | 200 mg |
| Methacrylic acid copolymer type C | 50 mg |
| Potassium bicarbonate | 343 mg |
| Potassium carbonate | 108 mg |
| Anhydrous citric acid | 384 mg |
| Fructose | 250 mg |
| Polysorbate 80 | 50 mg |
| Lemon flavour | 20 mg |
| Silica | 20 mg |
| Simethicone | 10 mg |

### Example 4 - Orodispersible granules

| | |
|---|---|
| PEA-OXA | 400 mg |
| Magnesium Butyrate | 600 mg |
| Glyceryl Palmitostearate | 120 mg |
| Sorbitol | 180 mg |
| Fructose | 180 mg |
| Polysorbate 80 | 10 mg |
| PVP K30 | 10 mg |
| Sucrose Palmitate | 20 mg |
| Steviol glycosides | 5 mg |
| Flavour | 50 mg |

### Example 5 - Paediatric chewable tablet

| | |
|---|---|
| PEA-OXA | 100 mg |
| Calcium Butyrate | 150 mg |
| Glyceryl Palmitostearate | 50 mg |
| Sorbitol | 81.5 mg |
| Fructose | 220 mg |
| Croscarmellose | 50 mg |
| Citric acid | 22.4 mg |
| Flavour | 6 mg |
| Magnesium stearate | 9 mg |
| Silicon dioxide | 9 mg |
| Sucrose Palmitate | 6 mg |
| Calcium Carbonate | 94.1 mg |
| Calcium Carbonate | 100 mg |

### Example 6 - Suppositories of 2.1 g

| | |
|---|---|
| Base for suppositories C10-18 | |
| Triglycerides-Polisorbato 65) | 1700 mg |
| Lysine Butyrate | 300 mg |
| PEA-OXA | 100 mg |

### Example 7 - Water-dispersible granules (sachet)

| | |
|---|---|
| Inulin | 2400 mg |
| Calcium Butyrate | 1800 mg |
| PEA-OXA | 1200 mg |
| Fructose | 400 mg |
| Calcium Carbonate | 200 mg |
| Citric Acid | 200 mg |
| Sorbitol | 180 mg |
| Glyceryl Dibehenate | 120 mg |
| Guar Gum | 100 mg |
| Polysorbate 80 | 20 mg |
| PVP K30 | 100 mg |
| Sucrose Palmitate | 20 mg |
| Silica | 100 mg |
| Flavour | 50 mg |
| Steviol glycosides | 7.5 mg |

### Example 8 - Soft gelatine capsule

| | |
|---|---|
| Bovine gelatine | 300 mg |
| PEA-OXA | 100 mg |
| Magnesium Butyrate | 150 mg |
| Glyceryl Palmitostearate | 80 mg |
| Corn oil | 400 mg |
| Glycerol | 125 mg |
| Sunflower lecithin | 30 mg |
| Water | 20 mg |
| Natural tocopherol | 10 mg |

### Example 9 - Hard gelatine capsules (A+B)

### Capsule A

| | |
|---|---|
| Sodium Butyrate | 500 mg |
| Hydroxypropylmethylcellulose | 140 mg |
| Glyceryl Palmitostearate | 50 mg |
| Silica | 20 mg |

### Capsule B

| | |
|---|---|
| PEA-OXA | 400 mg |
| Hydroxypropylmethylcellulose | 140 mg |
| Microcrystalline cellulose | 120 mg |
| Magnesium Carbonate | 40 mg |
| Magnesium Stearate | 10 mg |

### Example 10 - Water-dispersible granules (sachet)

| | |
|---|---|
| Inulin | 2400 mg |
| Calcium Butyrate | 1800 mg |
| PEA-OXA | 600 mg |
| Ultra-micronized PEA | 600 mg |
| Fructose | 400 mg |
| Calcium Carbonate | 200 mg |
| Citric Acid | 200 mg |
| Sorbitol | 180 mg |
| Glyceryl Dibehenate | 120 mg |
| Guar Gum | 100 mg |
| Polysorbate 80 | 20 mg |
| PVP K30 | 100 mg |
| Sucrose Palmitate | 20 mg |
| Silica | 100 mg |
| Flavour | 50 mg |
| Steviol glycosides | 7.5 mg |

### Example 11 - Hard gelatine capsules (A+B)

### Capsule A

| | |
|---|---|
| Sodium Butyrate | 500 mg |
| Hydroxypropylmethylcellulose | 140 mg |
| Glyceryl Palmitostearate | 50 mg |
| Silica | 20 mg |

### Capsule B

| | |
|---|---|
| PEA-OXA | 300 mg |
| PEA | 100 mg |
| Hydroxypropylmethylcellulose | 140 mg |
| Microcrystalline cellulose | 120 mg |
| Magnesium Carbonate | 40 mg |
| Magnesium Stearate | 10 mg |

### Example 12 - Orodispersible granules

| | |
|---|---|
| PEA-OXA | 300 mg |
| Micronized PEA | 100 mg |
| Magnesium Butyrate | 600 mg |
| Glyceryl Palmitostearate | 120 mg |
| Sorbitol | 180 mg |
| Fructose | 180 mg |
| Polysorbate 80 | 10 mg |
| PVP K30 | 10 mg |
| Sucrose Palmitate | 20 mg |
| Steviol glycosides | 5 mg |
| Flavour | 50 mg. |

## Claims

1. 2-Pentadecyl-2-oxazoline (PEA-OXA) for use in the prevention and treatment of epileptogenesis and epilepsy-associated comorbidities, wherein PEA-OXA is administered in association or in combination with butyric acid or a salt thereof, wherein said administration is separate, joint, or simultaneous.

2. 2-Pentadecyl-2-oxazoline for use according to claim 1, in the management of epileptic seizures.

3. 2-Pentadecyl-2-oxazoline for use according to claim 1 or 2, wherein, when the butyric acid is in the form of salt, said salt will preferably be selected from sodium salt, potassium salt, calcium salt, magnesium salt, lysine salt, arginine salt or a combinations thereof.

4. 2-Pentadecyl-2-oxazoline for use according to any one of claims 1 to 3, wherein PEA-OXA and butyric acid or a salt thereof are administered in a butyric acid or a salt thereof/PEA-OXA weight ratio between 1:1 and 7:1, preferably between 2:1 and 5:1, more preferably between 2.5:1 and 3.5:1, even more preferably of about 3:1.

5. 2-Pentadecyl-2-oxazoline for use according to any one of claims 1 to 4, wherein, both in a combination therapy and in a composition of PEA-OXA with butyric acid or a salt thereof, the daily dose of PEA-OXA will be at least between 100 mg/day and 1500 mg/day, preferably between 200 mg/day and 1200 mg/day, and the preferred daily dose of butyric acid or a salt thereof will be at least between 300 mg/day and 3500 mg/day, more preferably between 400 mg/day and 3000 mg/day.

6. 2-Pentadecyl-2-oxazoline for use according to any one of claims 1 to 5, wherein 2-pentadecyl-2-oxazoline and butyric acid or a salt thereof exert a synergistic effect.

7. A pharmaceutical or veterinary composition comprising PEA-OXA and butyric acid or a salt thereof, wherein PEA-OXA and butyric acid or a salt thereof are present in a butyric acid or a salt thereof/PEA-OXA weight ratio between 1:1 and 7:1, preferably between 2:1 and 5:1, more preferably between 2.5:1 and 3.5:1, even more preferably of about 3:1, and wherein the amount of PEA-OXA per dose unit is between 100 mg and 1500 mg, preferably between 200 mg and 1200 mg.

8. The composition according to claim 7, further comprising palmitoylethanolamide (PEA) in amounts between 100 mg and 1500 mg per dose unit.

9. A dietary composition, food supplement, complementary feed and food for special medical purposes (FSMP) comprising PEA-OXA and butyric acid or a salt thereof, wherein PEA-OXA and butyric acid or a salt thereof are present in a butyric acid or a salt thereof/PEA-OXA weight ratio between 1:1 and 7:1, preferably between 2:1 and 5:1, more preferably between 2.5:1 and 3.5:1, even more preferably of about 3:1, and wherein the amount of PEA-OXA per dose unit is between 100 mg and 1500 mg, preferably between 200 mg and 1200 mg.
